# EUROPEAN PATENT APPLICATION

(11) **EP 1 617 200 A1**
(43) Date of publication of application: **18.01.2006**
(21) Application number: 05077148.4
(22) Date of filing: 08.07.1999
(51) Int. Cl.: G01N 1/00, G01N 33/00, G01N 33/18

(54) **Kit for measuring volatile organic compounds**

(30) Priority: 08.07.1998 US 92309 P; 27.08.1998 US 98135 P
(62) Divisional of application: 99951371.6
(71) Applicant: Midwest Research Institute, Kansas City MO 64110-2299 (US)
(72) Inventor: The designation of the inventor has not yet been filed
(74) Representative: Hartley, Andrew Philip

(57) **Abstract**

A kit is provided for measuring the volatile organic compounds of a material produced in a process system having emissions. The kit comprises an enclosed bag (10) having a sealable opening to allow an amount of said material to be placed in said enclosed bag so that there is headspace above said material. The kit also includes instructions for analyzing samples from said headspace in said enclosed bag, thereby providing said volatile organic compounds of such material. The bag is designed to be vapour impermeable.

## Description

### FIELD OF THE INVENTION

The present invention generally relates to measuring volatile organic compounds. More particularly, the invention relates to a convenient, inexpensive, quick method by which the volatile organic compounds (VOC) of a material produced in a process system can be measured and a kit containing an enclosed bag with a sealable opening and instructions for the VOC measurement method.

### BACKGROUND OF THE INVENTION

A long-standing problem in the chemically-related manufacturing industry has been the way in which the rate of VOC emissions is controlled and monitored. The concerns associated with VOC control and monitoring are well rooted in governmental policies throughout the world, all of which arc aimed at reducing the emission of such VOCs into the atmosphere. Additionally, the manufacturing industries themselves have been notably concerned with safety and environmental concerns associated with VOC emissions. As a result, since the onset of the industrial revolution, the chemically-related manufacturing industry has striven for zero to minimal VOC emissions. To that end, relatively expensive and time-consuming VOC measurement techniques have been developed and have been constantly employed to monitor VOC emissions of virtually every unit operation in every manufacturing facility throughout the world. In fact, numerous companies have sprouted into existence which specialize in testing techniques for VOCs and aid in ensuring compliance with specific strict company as well as governmental regulations. Such specialization and expertise render these services extremely expensive, and therefore, significantly add to the overall expense of whatever product is being manufactured. Accordingly, there remains a need in the art for an inexpensive, less time-consuming, method by which VOCs can be conveniently measured for a given material being produced in a process system.

### SUMMARY OF THE INVENTION

The invention meets the needs in the art by providing a method for measuring VOCs of a material produced in a process system or unit operation. The method involves an enclosed bag into which a sample of material is placed, after which the bag is stored at a predetermined temperature such that the contents reach equilibrium. The storage temperature is the mean exit temperature of the effluent from the process system for which a VOC measurement is required. Samples from the headspace in the bag are inputted into a Flame Ionization Detector or equivalent apparatus to provide the VOC level.

In accordance with one aspect of the invention, a method for the measuring volatile organic compounds of a material produced in a system having emissions or an effluent is provided. The method comprises the steps of: (a) disposing an amount of the material in an enclosed bag having a sealable opening such that there is headspace above the material in the enclosed bag; (b) storing the enclosed bag containing the solid material at the mean exit temperature of the emissions of the system such that equilibrium between the material and the headspace is reached; and (c) introducing samples from the headspace into a flame ionization detector which thereby measures the volatile organic compounds of the material.

In another aspect of the invention, a kit for measuring the volatile organic compounds of a material produced in a system having emissions is provided. The kit comprises: (a) an enclosed bag having a sealable opening to allow an amount of said material to be placed in said enclosed bag such there is headspace above said material; and (b) instructions for analyzing samples from said headspace in said enclosed bag, thereby providing said volatile organic compounds of said material. In preferred modes, the instructions for analyzing samples include withdrawing said samples from said headspace using a flame ionization detector, and/or storing the enclosed bag in a temperature adjustable apparatus.

All percentages and proportions herein are by weight, and all references cited are hereby incorporated by reference, unless otherwise specifically indicated.

Accordingly, it is an advantage of the invention to provide an inexpensive, less time-consuming, method by which VOCs can be conveniently measured for a given material being produced in a process system. This and other features and attendant advantages of the present invention will become apparent to those skilled in the art from a reading of the following detailed description of the preferred embodiment, drawing and the appended claims.

### BRIEF DESCRIPTION OF THE DRAWINGS

Fig. 1 is a side-view of an enclosed bag used in the VOC measurement method described herein; and
Fig. 2 is a graphical representation of the data generated in Example II for determining the minimum amount of sample required to obtain an accurate VOC measurement.

### DETAILED DESCRIPTION OF THE PREFERRED EMBODIMENT

The method in accordance with the invention essentially requires the use of an enclosed bag, and a means for chemically analyzing samples from the headspace inside the bag. The enclosed bag preferably has a sealable opening so that a sample of material can be placed inside the bag, after which it is sealed for storage. After storage, the bag is unsealed so that samples (typically vapor) from the headspace can be taken for chemical analysis. As used herein, the "material" for which the VOCs are required can be a liquid, paste or solid. The method disclosed herein has several advantages in that it only requires a small sample for determining the VOC of a large process system without using expensive, on-line (i.e., moving the equipment to the VOC discharge point) analytical equipment, and the method provides an early and convenient VOC emission indication. The latter advantage being extremely useful for researchers experimenting with new raw materials and need to know the potential downstream VOC emissions of such raw materials when ultimately scaled-up to commercial production volumes.

In typical situations in which a VOC measurement is required, a bench-scale or commercial-scale process system is in place, and for any give unit operation within such a process system, an effluent is generated which typically comprises air or other gas containing VOCs. It is the VOC content in this effluent resulting from the production of the material in the unit operation or process system that is measured by the method described herein. By way of example, in the commercial manufacture of laundry detergents, it has been conventional for fluid bed dryers to be used to produce granular detergent products. In such a process system, the granules produced vary in composition. Consequently, the amount of VOCs produced in such detergent manufacturing operations involving fluid bed dryers will vary accordingly.

This method can be conveniently employed to measure the VOCs of a fluid bed drying operation for the production of a granular detergent product having a particular composition. If the composition is changed as is quite often the case with commercial detergent manufacturing, the method can be easily and quickly employed to determine the new VOC amount. In such an operation, a sample (5 to 100 grams) of the granules from the fluid bed dryer are sampled and placed in an enclosed bag (e.g., 7400 cc). The enclosed bag preferably has an inner liner formed of aluminum foil and an outer liner of polymeric material (e.g., polyethylene) such that the bag can be collapsible while samples are being withdrawn from the headspace. The foil lining can be made of material other than foil so long as it achieves the purpose of being vapor impermeable so as not to lead to inaccurate VOC measurements. It should be understood, however, that any bag is suitable for use herein so long as it can be safely stored at the requisite temperature and remain sufficiently collapsible for the samples to be withdrawn from the headspace above the sample for Flame Ionization Detection (FID) and does not contribute to the VOC measurement. The mean exit temperature of the effluent from the fluid bed dryer is determined so that a conventional oven can be set to that temperature. The bag is then placed in the oven at the mean exit temperature of the fluid bed dryer for a time sufficient for the contents to reach equilibrium.

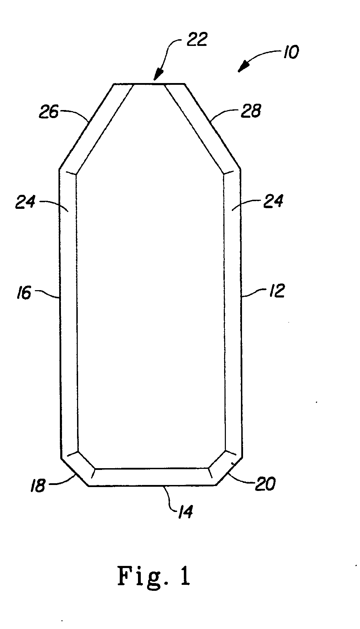

Referring now to Fig. 1, an enclosed bag 10 in accordance with the invention is shown form a side-view. The bag 10 can be easily constructed by a two layer (inner foil layer and outer polymer layer) 100 cm x 35 cm sheet and folding it in half, then folding three edges 12, 14, 16 and the four comers, 18, 20, 26 and 28 to form a the bag 10 having a conical an opening 22 through which the samples are placed into the bag 10. The bag 10 edges 12, 14 and 16 and corners 18, 20, 26 and 28 can be sealed with heavy duty duct tape 24 or equivalent sealing means. The resulting bag 10 volume is about 7400 cm³. Those skilled in the art will appreciate the numerous other ways in which similar bags can be constructed for use in the method invention described herein.

While not intending to be bound by theory, it is theorized that in real process or unit operation systems such as a fluid bed dryer, significant amounts of hot air removes water from the material for which a VOC measurement is needed. While the water is being removed, VOCs are also transported out of the material due to the increase in material temperature as drying or other process operation proceeds. Estimating the amount of VOC using a mathematical model is difficult because of the amount of information required, including the organic composition of the material, amount of organics in the material, vapor pressure of the material, amount of diffusion of organics through the material, mass transfer of organics from the surface of the material, temperature of the system, and the equilibrium concentration above or in the headspace of the material. The present method provides a convenient direct measurement of the equilibrium concentration above the material, i.e., in the headspace in the bag above the sample material.

Exemplary process systems or unit operations on which the method may be employed, include but are not limited to, spray dryers, mixers, fluid bed dryers and coolers, and storage tanks. The storage times will vary widely depending upon the process system and material for which a VOC measurement is needed, but typical times are for from about 1 hour to about 3 days, more typically from about 5 hours to about 24 hours. Exemplary sample amounts will depend on the bag size, but typically is from about I gram to about 100 grams. The minimum amount of sample material can be determined as described in Example II hereinafter. The amount will vary as mentioned previously, but is generally determined by using a sample amount that ensures that equilibrium has been reached between the sample and the headspace inside the bag. In certain process systems or unit operations, true equilibrium between the volatile organic compounds and the process system may not occur for a variety of reasons, for example the process may have a short cycle time. In these situations, the present process can be used to obtain the maximum VOC amount at a given temperature of the process system as the true VOC will be some fraction of the VOC value determined by the present process. Similarly, the storage temperatures will vary widely depending upon the mean exit temperature of the process system, but a typical mean exit is from about 5°C to about 100°C.

A commercial product which can be sold specifically for using the method of the invention can be in the form of a kit for measuring the volatile organic compounds of a material produced in a system having emissions. The kit may include the bag having a sealable opening to allow an amount of the material to be placed in the enclosed bag such there is headspace above the material, and a means for analyzing samples from the headspace in the enclosed bag such as a FID. The kit will preferably include instructions for use which generally tracks the method described herein.

The following examples are intended to illustrate one or more of the various embodiments of the invention, but are not intended to be limiting in any manner of the full scope of the claims appended hereto.

### EXAMPLE I

A bag suitable for use in the method is constructed or obtained as described with respect to the Figure. In a large-scale manufacturing facility in which a detergent composition is being produced, a large sample of detergent particles being dried in a fluid bed dryer is taken at the inlet of the dryer and riffled down to five 20 gram samples. The bags are constructed of material composed of 100% 28 gauge aluminum foil on the inside (for sterile/inert cavity) and 48 gauge polyethylene on the outside (for strength). The bag material is cut into a 100 cm by 35 cm rectangle and folded with the aluminum on the inside. The sides of the bag are folded with two approx. 6 mm folds and the open end is folded on both sides with 10 mm folds until there is only approx. a 40 mm opening left resulting in a angled/cone shaped appearance. All folds are then sealed with high strength/high temperature fabric or duct tape leaving only the 40 mm opening unsealed. Each of the five 20 gram samples are poured into separate bags as shown in the Figure. Thereafter, each bag is filled with ultra high purity air (0% hydrocarbon content) to about 90% of the expanded volume to leave room for taping the bag opening closed (opening folded twice and tape shut) and for bag expansion due to eventual heating of the bag. The mean exit temperature of the fluid bed dryer is 120°F (49°C), and therefore, the enclosed bags are stored in a constant temperature oven at 120°F (49°C) for 12 hours to allow the headspace in each bag to come into equilibrium with the sample. Thereafter, each of the five samples are withdrawn from the bag using a probe which is fed into a Flame Ionization Detector ("FID"; commercially available from Eagle Monitoring Systems, Inc., Model No. EM 7000). Conventional operation instructions for the FID are followed which include but is not limited to the following steps:
1) Setup - Install regulators on cylinders and connect to proper inlet ports on the EM 7000. Use 1/4" TPFE teflon tubing. If data taker is being used connect wires to 4 - 20 mA located on back of EM 7000. Plug EM 7000 into 120 V outlet, power up EM 7000 by depressing the red button on the front panel in this position the power switch light will be lit, also the ignition switch (yellow) light will be on. This is to indicate a "flame out" condition. Depress the blue buttons for desired range (0-100, 0-1000, 0-10,000, 0-100,000 ppm). Turn on the oven to heat up cell, button is marked oven located on front panel. Temperature controller is located in the center of the front panel. Set point should be set at 150°C oven will take approximately 1 hour to heat to this temperature. When oven is almost to temperature turn on pump, button is located on the front panel, also turn on controller for the heated sample hose. Open valves to gas cylinders, set the regulators for fuel and combustion air to 40 psi. Set rotameters located on the front panel of FID, rotameter labeled fuel is set at approximately 22 cc/min., rotameter labeled combustion air is set at approximately 200 cc/min. Set regulators for zero and calibration gases to 40 psi.; there is a single stage regulator attached to reduce the pressure from 40 psi. to no more than 1 psi. Fuel and combustion air needs to flow through unit for approximately 15 mins. before lighting flame.
2) Ignition of flame - A 2-position switch located on the back panel is used to select one of two glow plugs used to ignite the flame. Depress the ignition switch and hold no longer than 20 seconds.
3) Calibration - Select the low range button, and make sure EM 7000 is stable at the desired temperature. Adjust sample back pressure. This is done by turning the black knob on the front panel of the unit until pressure gauge located on the front panel is at the specified setting. The specified setting for sample back pressure is 2.5 psi.. Ensure that the flow rate of H₂ and combustion air are at the specified setting (the flow rates are 22 cc/min. for H₂ and 200 cc/min. for combustion air). Introduce zero air to the instrument by depressing the "zero" button. Adjust sample back pressure to 2.5 psi.
   Observe the baseline on the data recorder, and adjust the zero potentiometer until display reads approximately zero ppm.
4) Calibration adjustment procedure - Select the calibration range by depressing the appropriate range button. Introduce calibration gas to the analyzer by selecting the span button, and adjust sample back pressure to 2.5 psi. Observe the concentrations of ppm on the data recorder and adjust the calibration (gain) potentiometer on the front panel to set the meter to read the value of the calibration gas as indicated on the cylinder. Depress the "span" button and check zero again, adjust if necessary (remember to set sample back pressure to 2.5 psi), recheck calibration and adjust if necessary. This may have to be repeated three or four times. Depress both span and zero buttons; the instrument is now pulling in sample gases. Adjust sample back pressure to 2.5 psi.

The VOCs of each of the five samples contained in the bag are averaged to give a value of 15 ppm by volume on a propane ppm basis. Unexpectedly, this VOC measurement value is statistically the same as measurements taken using much more expensive, time-consuming VOC on-line measurement equipment.

### EXAMPLE II

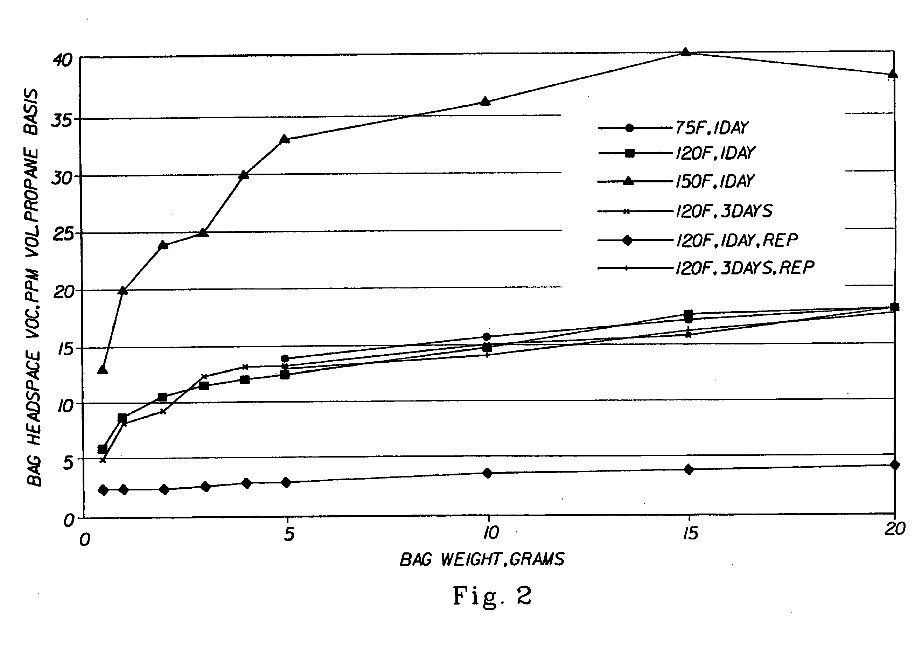

This Example sets forth a method by which the minimum amount of sample material necessary for ensuring accuracy of the VOC measurement can be determined. This is based on determining the equilibrium level inside the bag for a given unit operation (e.g., fluid bed dryer) operating at a certain temperature. Specifically the following steps are conducted:
1. Place 0.5 grams of a sample material in a bag as described in Example I;
2. Place the bag in an oven at a given temperature for 16 hours;
3. Sample the bag using a probe from a FID to measure the VOC in the headspace of the bag as described in Example I;
4. Repeat steps 1-3 using increasing amounts of a sample material;
5. Repeat steps 1-4 at different oven temperatures;
6. Repeat steps 1-5 at different storage times;
7. With the data collected from steps 4-6, generate a graph as shown in Fig. 2 (REP = repeat);
8. Using the graph from step 7 such as Fig. 2, the minimum amount of sample can be seen in that the flat part of the curves represents that equilibrium has been reached; any sample amount in the flat part of a given temperature curve will ensure an accurate VOC measurement; and
9. Use at least the sample amount for measuring the VOCs as described in Example 1; 20 grams is an appropriate amount based on Fig. 2 for a fluid bed system as exemplified in Example I. As the skilled artisan will appreciate, the methodology of steps 1-9 can be done on different systems (e.g., spray drying tower) and on liquid or paste samples in which the minimum sample amount may be substantially less than 20 grams.

Accordingly, having thus described the invention in detail, it will be obvious to those skilled in the art that various changes may be made without departing from the scope of the invention, and the invention is not to be considered limited to what is described in the specification.

Aspects of the invention are defined in the following clauses (which are not claims).
A) A method for the measuring volatile organic compounds of a material produced in a system having emissions, said method comprising the steps of:
   (a) disposing an amount of said material in an enclosed bag having a sealable opening such that there is headspace above said material in said enclosed bag;
   (b) storing said enclosed bag containing said solid material at the mean exit temperature of said emissions of said system such that equilibrium between said material and said headspace is reached; and
   (c) introducing samples from said headspace into a flame ionization detector which thereby measures said volatile organic compounds of said material.
B) The method of Clause A wherein said system is a fluid bed dryer.
C) The method of Clause A wherein said system is a spray dryer.
D) The method of Clause A wherein said storing step is for from about 5 hours to about 24 hours.
E) The method of Clause A wherein said amount of said material is from about 1 gram to about 100 grams.
F) The method of Clause A wherein said system is a storage tank.
G) The method of Clause A wherein said mean exit temperature is from about 5°C to about 100°C.
H) A kit for measuring the volatile organic compounds of a material produced in a system having emissions, said kit comprising:
   (a) an enclosed bag having a sealable opening to allow an amount of said material to be placed in said enclosed bag such there is headspace above said material; and
   (b) instructions for analyzing samples from said headspace in said enclosed bag, thereby providing said volatile organic compounds of said material.
I) The kit of Clause H wherein said instructions for analyzing samples include withdrawing said samples from said headspace using a flame ionization detector.
J) The kit of Clause H wherein said instructions for analyzing samples include storing said enclosed bag in a temperature adjustable apparatus.

## Claims

1. A kit for measuring the volatile organic compounds of a material produced in a process system having emissions, said kit comprising:
(a) an enclosed bag having a sealable opening to allow an amount of said material to be placed in said enclosed bag such that there is headspace above said material; and
(b) instructions for analyzing samples from said headspace in said enclosed bag, thereby providing said volatile organic compounds of said material;
wherein said bag is desired to be vapour impermeable.

2. The kit of claim 1 wherein said instructions for analyzing samples include withdrawing said samples from said headspace using a flame ionization detector.

3. The kit of claim 1 wherein said instructions for analyzing samples include storing said enclosed bag in a temperature adjustable apparatus.

4. The kit of claim 1 wherein said bag comprises an inner layer.

5. The kit of claim 4 wherein said inner layer comprises aluminium foil.

6. The kit of claim 5 wherein said aluminium foil is 100% 28 gauge aluminium foil.

7. The kit of claim 4 wherein said bag additionally comprises an outer layer of polyethylene.

8. The kit of claim 7 wherein said polyethylene comprises 48 gauge polyethylene.

9. The kit of claim 4 wherein said instructions for analyzing said samples include withdrawing said samples from said headspace using a flame ionization detector.

10. The kit of claim 4 wherein said instructions for analyzing samples include storing said enclosed bag in a temperature adjustable apparatus.
